(19) Europäisches Patentamt
European Patent Office
Office européen des brevets

(11) Veröffentlichungsnummer: **0 133 418**
**A2**


# (12) EUROPÄISCHE PATENTANMELDUNG


(21) Anmeldenummer: **84810381.8**

(22) Anmeldetag: **03.08.84**

(51) Int. Cl.[4]: **A 01 N 43/80**, C 07 D 275/04

(30) Priorität: **09.08.83 CH 4335/83**

(43) Veröffentlichungstag der Anmeldung: **20.02.85**
**Patentblatt 85/8**

(84) Benannte Vertragsstaaten: **BE CH DE FR GB IT LI NL**



(71) Anmelder: **CIBA-GEIGY AG, Postfach, CH-4002 Basel (CH)**

(72) Erfinder: **Drabek, Jozef, Dr., Benkenstrasse 12, CH-4104 Oberwil (CH)**


## (54) Die Verwendung von 3-Acylaminobenzisothiazolen in der Schädlingsbekämpfung.


(57) Die Verwendung in der Schädlingsbekämpfung von 3-Acrylamino-benzisothiazolen der Formel I

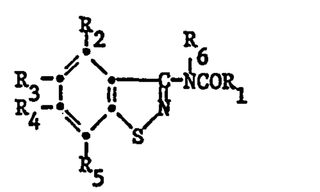

(I)

worin $R_1$ gegebenenfalls substituiertes Alkyl- oder Phenyl, $R_2$, $R_3$, $R_4$ und $R_5$ unabhängig voneinander Wasserstoff, Halogen, $C_1$-$C_5$-Alkyl, $C_1$-$C_5$-Alkoxy, Trifluormethyl, Amino oder Nitro und $R_6$ Wasserstoff oder $-COR_1$ bedeuten.

Ebenso werden die neuen Verbindungen der Formel Ia

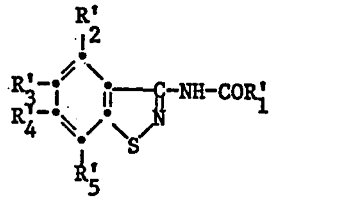

(Ia)

worin $R'_1$ durch Halogen oder $C_1$-$C_5$-Alkoxy substituiertes $C_1$-$C_{10}$ Alkyl oder unsubstituiertes $C_2$-$C_{10}$-Alkyl oder Phenyl und

$R'_2$, $R'_3$, $R'_4$ und $R'_5$ unabhängig voneinander Wasserstoff, Halogen, $C_1$-$C_5$-Alkyl, $C_1$-$C_5$-Alkoxy, Trifluormethyl, Amino oder Nitro und $R'_6$ Wasserstoff oder $-COR'_1$ oder

$R'_1$ Methyl, $R'_2$, $R'_3$, $R'_4$ und $R'_5$ unabhängig voneinander Wasserstoff, Halogen, $C_1$-$C_5$-Alkyl, $C_1$-$C_5$-Alkoxy, Trifluormethyl, Amino oder Nitro und $R'_6$ Wasserstoff oder

$R'_1$ Methyl, $R'_2$ Wasserstoff, $C_1$-$C_5$-Alkyl, $C_1$-$C_5$-Alkoxy, Trifluormethyl, Amino oder Nitro und $R'_6$ Wasserstoff oder

$R'_1$ Methyl, $R'_2$ Wasserstoff, $C_1$-$C_5$-Alkyl, $C_1$-$C_5$-Alkoxy, Trifluormethyl, Amino oder Nitro, $R'_3$, $R'_4$ und $R'_5$ unabhängig voneinander Wasserstoff, Trifluormethyl, Amino oder Nitro und $R'_6$-$COR'_1$ bedeuten und Schädlingsbekämpfungsmittel, welche als aktive Komponente Verbindungen der Formeln I bzw. Ia enthalten sowie Verfahren zur Herstellung dieser 2-Acylaminobenzisothiazole beschrieben.


EP 0 133 418 A2

CIBA-GEIGY AG 5-14531/+

Basel (Schweiz)

Die Verwendung von 3-Acylaminobenzisothiazolen in der Schädlingsbekämpfung.

Die vorliegende Erfindung betrifft die Verwendung von 3-Acylaminobenzisothiazolen in der Schädlingsbekämpfung, sowie Mittel, welche als aktive Komponente solche 3-Acylaminobenzisothiazole enthalten, die neuen 3-Acylaminobenzisothiazole und Verfahren zu ihrer Herstellung. Die erfindungsgemäss zu verwendenden 3-Acylaminobenzisothiazole haben die Formel

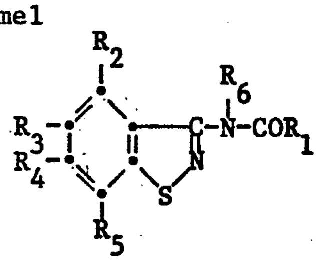

(I)

worin $R_1$ gegebenenfalls substituiertes Alkyl- oder Phenyl, $R_2$, $R_3$, $R_4$ und $R_5$ unabhängig voneinander Wasserstoff, Halogen, $C_1$-$C_5$-Alkyl, $C_1$-$C_5$-Alkoxy, Trifluormethyl, Amino oder Nitro und $R_6$ Wasserstoff oder $-COR_1$ bedeuten.

Die Verbindungen der Formel I können auch in ihrer tautomeren Form der Formel

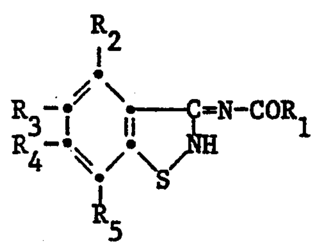

vorliegen, falls $R_6$ Wasserstoff bedeutet.

Unter Halogen ist dabei Fluor, Chlor, Brom oder Jod, insbesondere aber Chlor oder Fluor, zu verstehen.

Die bei $R_1$ bis $R_5$ in Frage kommenden Alkyl- und Alkoxygruppen können geradkettig oder verzweigt sein. Die Alkylgruppen bei $R_1$ haben 1 bis 10, bevorzugt 1 bis 5, Kohlenstoffatome.

Beispiele von Substituenten an den Alkyl- und Phenylgruppen bei $R_1$ sind u.a.: Halogen, $C_1$-$C_5$-Alkoxy, Amino, Nitro und/oder Trifluormethyl.

Beispiele von Alkoxy- und gegebenenfalls substituierten Alkylgruppen bei $R_1$ bis $R_5$ sind u.a.:
Methyl, Methoxy, $-CH_2Cl$, Trifluormethyl, Aethyl, Aethoxy, $-CH_2CH_2F$, $-CF_2CF_3$, Propyl, $-CF_2-CF_2-CF_3$, Isopropyl, n-, i-, sek.-, tert.-Butyl, n-Pentyl, n-Hexyl, n-Decyl und deren Isomere.

Die Verbindung der Formel

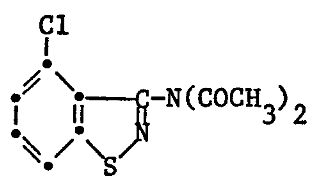

ist in der DOS Nr. 1,915,387 als Herbizid beschrieben.

Bevorzugt sind die neuen Verbindungen der Formel Ia

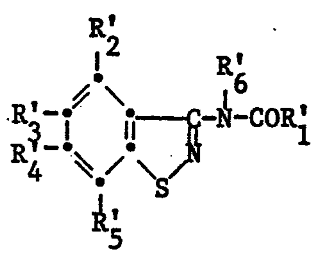

(Ia)

worin $R'_1$ durch Halogen oder $C_1$-$C_5$-Alkoxy substituiertes $C_1$-$C_{10}$Alkyl oder unsubstituiertes $C_2$-$C_{10}$-Alkyl oder Phenyl,
$R'_2$, $R'_3$, $R'_4$ und $R'_5$ unabhängig voneinander Wasserstoff, Halogen, $C_1$-$C_5$-Alkyl, $C_1$-$C_5$-Alkoxy, Trifluormethyl, Amino oder Nitro und

$R'_6$ Wasserstoff oder $-COR'_1$ oder
$R'_1$ Methyl, $R'_2$, $R'_3$, $R'_4$ und $R'_5$ unabhängig voneinander Wasserstoff, Halogen, $C_1$-$C_5$-Alkyl, $C_1$-$C_5$-Alkoxy, Trifluormethyl, Amino oder Nitro und $R'_6$ Wasserstoff oder
$R'_1$ Methyl, $R'_2$ Wasserstoff, $C_1$-$C_5$-Alkyl, $C_1$-$C_5$-Alkoxy, Trifluormethyl, Amino oder Nitro, $R'_3$, $R'_4$ und $R'_5$ unabhängig voneinander Wasserstoff, Trifluormethyl, Amino oder Nitro und $R'_6$ $-COR'_1$ bedeuten.

Insbesondere bevorzugt sind Verbindungen der Formel Ia, worin $R'_1$ durch Fluor oder Chlor substituiertes $C_1$-$C_5$-Alkyl oder unsubstituiertes $C_2$-$C_5$-Alkyl,
$R'_2$, $R'_3$, $R'_4$ und $R'_5$ unabhängig voneinander Wasserstoff, Chlor, Methyl, Trifluormethyl, Amino oder Nitro und $R'_6$ Wasserstoff oder $-COR'_1$ oder
$R'_1$ Methyl,
$R'_2$, $R'_3$, $R'_4$ und $R'_5$ unabhängig voneinander Wasserstoff, Chlor, Methyl, Trifluormethyl, Amino oder Nitro und $R'_6$ Wasserstoff oder
$R'_1$ Methyl, $R'_2$ Wasserstoff, $C_1$-$C_5$-Alkyl, $C_1$-$C_5$-Alkoxy, Trifluormethyl, Amino oder Nitro, $R'_3$, $R'_4$ und $R'_5$ unabhängig voneinander Wasserstoff, Chlor, Methyl, Trifluormethyl, Amino oder Nitro und $R'_6$ $-COR_1$ bedeuten.

Die Verbindungen der Formel Ia, welche auch in ihrer tautomeren Form der Formel

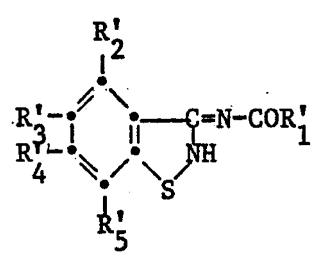

vorliegen können (falls $R'_6$ = Wasserstoff), werden nach an sich bekannten Methoden z.B. wie folgt hergestellt:

1.

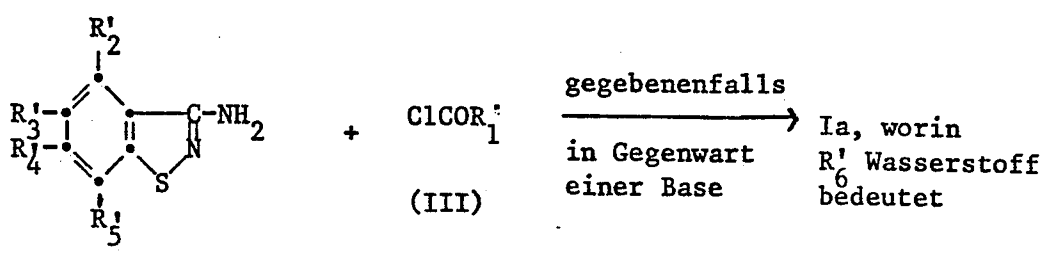


(II)

2. 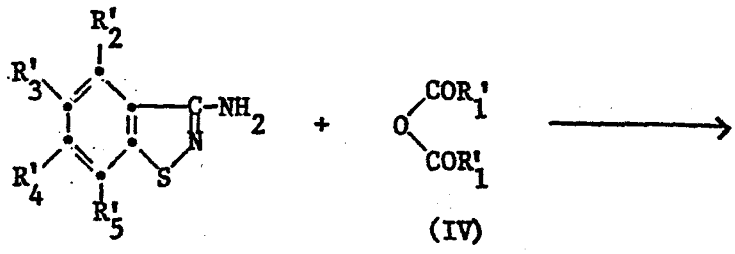

Ia, worin $R'_6$ $COR'_1$ bedeutet

(IV)

(II)

In den Formeln II, III und IV haben $R'_1$ bis $R'_5$ die für die Formel Ia angegebene Bedeutung. Auf analoge Weise werden auch die Verbindungen der Formel I hergestellt.

Als geeignete Basen kommen insbesondere tertiäre Amine wie Trialkylamine, Dialkylaniline und p-Dialkylaminopyridine in Betracht.

Die Verfahren werden bei normalem Druck, bei einer Temperatur von -25° bis 150°C, vorzugsweise bei 50° bis 100°C und gegebenenfalls in einem Lösungs- oder Verdünnungsmittel durchgeführt.

Als Lösungs- oder Verdünnungsmittel eignen sich z.B. Aether und ätherartige Verbindungen wie Diäthyläther, Diisopropyläther, Dioxan und Tetrahydrofuran; aromatische Kohlenwasserstoffe wie Benzol, Toluol und Xylole; Ketone wie Aceton, Methyläthylketon und Cyclohexanon; Nitrile wie Acetonitril; Ester wie Aethylacetat und Butylacetat; sowie Dimethylformamid, Dimethylsulfoxid, Methylcyanid und halogenierte Kohlenwasserstoffe.

Die Verbindungen der Formel II können auch in ihrer tautomeren Form der Formel

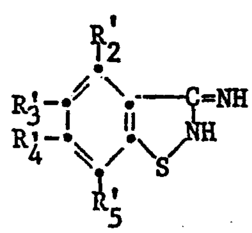

vorliegen.

Die Ausgangsstoffe der Formeln II, III und IV sind bekannt oder können nach bekannten Verfahren hergestellt werden.

Die Verbindungen der Formel I bzw. Ia eignen sich zur Bekämpfung von Schädlingen an Tieren und Pflanzen sowie im Boden.

Insbesondere eignen sich die Verbindungen der Formel I bzw. Ia zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von Milben und Zecken der Ordnung Akarina.

Vor allem eignen sich Verbindungen der Formel I bzw. Ia zur Bekämpfung von pflanzenschädigenden Insekten, insbesondere pflanzenschädigenden Frassinsekten, in Zier- und Nutzpflanzen, insbesondere in Baumwoll- und Reiskulturen (z.B. Spodoptera littoralis, Heliothis virescens, Chilo suppressalis und Laodelphax) sowie Gemüse- und Obstkulturen (z.B. Leptinotarsa decemlineata, Myzus persicae, Laspeyresia pomonella und Adoxophyes reticulana) und von Bodeninsekten (z.B. Aulacophora femoralis, Chortophila brassicae, Diabrotica balteata, Pachnoda savigni und Scotia ypsilon).

Wirkstoffe der Formel I bzw. Ia zeigen auch eine sehr günstige Wirkung gegen Fliegen, wie z.B. Musca domestica und Mückenlarven.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; andere pyrethrinartige Verbindungen sowie Karbamate und chlorierte Kohlenwasserstoffe.

Mit besonderem Vorteil werden Verbindungen der Formel I bzw. Ia auch mit Substanzen kombiniert, welche einen synergistischen oder verstärkenden Effekt auf Pyrethroide ausüben. Beispiele solcher Verbindungen sind u.a. Piperonylbutoxid, Propionyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithionate, 1,2-Methylendioxy-4-(2-(octylsulfinyl)-propyl)-benzol.

Die Verbindungen der Formel I bzw. Ia werden in unveränderter Form oder vorzugsweise zusmman mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I bzw. Ia und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder

Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I bzw. Ia nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie

z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5 Aethylenglykoleinheiten.

Als Beispiele nichtionischer Tenside seien Nonylphenolpolyäthoxyäthanole, Ricinusölpolyglykoläther, Polypropylenpolyäthylenoxid-Addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi-(2-chloräthyl)-äthylammoniumbromid.

Die in der Formulierungstechnik gebräuchlichen Tenside sind u.a. in folgenden Publikationen beschrieben:
"Mc Cutcheon's Detergents and Emulsifiers Annual" MC Publishing Corp., Ridgewood, New Jersey, 1982; Dr. Helmut Stache: "Tensid Taschenbuch" Carl Hanser Verlag München/Wien 1981.

Die pestiziden Zubereitungen enthalten in der Regel 0,1 bis 99 %, insbesondere 0,1 bis 95 %, Wirkstoff der Formel I bzw. Ia, 1 bis 99,9% eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe der Formel I bzw. Ia (% = Gewichtsprozent)

| 1. Emulsions-Konzentrate | a) | b) | c) |
|---|---|---|---|
| Wirkstoff | 25% | 40% | 50% |
| Ca-Dodecylbenzolsulfonat | 5% | 8% | 6% |
| Ricinusölpolyäthylenglykoläther (36 Mol AeO) | 5% | - | - |
| Tributylphenolpolyäthylenglykoläther (30 Mol AeO) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 2. Granulate | a) | b) |
|---|---|---|
| Wirkstoff | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| 3. Stäubemittel | a) | b) |
|---|---|---|
| Wirkstoff | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Formulierungsbeispiele für feste Wirkstoffe der Formel I bzw. Ia (% = Gewichtsprozent)

| 4. Spritzpulver | a) | b) | c) |
|---|---|---|---|
| Wirkstoff | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyäthylenglykol-äther (7-8 Mol AeO) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 5. Emulsions-Konzentrat | |
|---|---|
| Wirkstoff | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 6. Stäubemittel | a) | b) |
| --- | --- | --- |
| Wirkstoff | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

| 7. Extruder Granulat | |
| --- | --- |
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

| 8. Umhüllungs-Granulat | |
| --- | --- |
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

9. Suspensions-Konzentrat

| | |
|---|---|
| Wirkstoff | 40% |
| Aethylenglykol | 10% |
| Nonylphenolpolyäthylenglykol-äther (15 Mol AeO) | 6 % |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1: Herstellung von 3-Diacetylaminobenzisothiazol

Ein Gemisch bestehend aus 11,7 g 3-Aminobenzisothiazol, 126 ml Acetanhydrid und 0,2 g Natriumacetat wird 48 Stunden lang bei 100 - 110°C gerührt. Nach dem Einengen und Chromatographieren (Kieselsäure; Eluiermittel Hexan-Diäthyläther 2:1) wird das Produkt aus Hexan-Diäthyläther umkristallisiert. Man erhält die Verbindung Nr. 1 der Formel

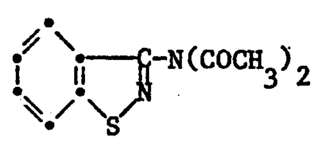

mit einem Schmelzpunkt von 65-67°C.

Auf analoge Weise werden auch folgende Verbindungen hergestellt:

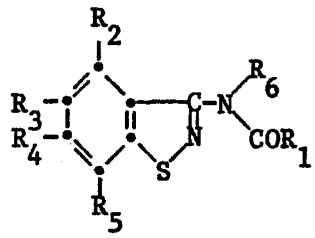

| Nr. | $R_1$ | $R_2$ | $R_3$ | $R_4$ | $R_5$ | $R_6$ | Physikalische Daten |
|---|---|---|---|---|---|---|---|
| 2 | $-CH_3$ | Cl | H | H | H | $-COCH_3$ | Smp.:107-109°C |
| 3 | $-CH_3$ | Cl | H | H | $-NO_2$ | H | Smp.:193°C |
| 4 | $-C_3H_{7(n)}$ | Cl | H | H | H | $-COC_3H_{7(n)}$ | Smp.:76-80°C |
| 5 | $-C_2H_5$ | Cl | H | H | H | $-COC_2H_5$ | Smp.:101-103°C |
| 6 | $-CH_2Cl$ | Cl | H | H | H | $-COCH_2Cl$ | Smp.:118-119°C |
| 7 | $-CF_2CF_3$ | Cl | H | H | H | H | Smp.: 98-99°C |
| 8 | $-CF_3$ | Cl | H | H | H | H | Smp.:127-129°C |
| 9 | $-CF_2CF_2OF_3$ | Cl | H | H | H | H | Smp.:97-98°C |
| 10 | $-CF_3$ | $-CH_3$ | H | H | H | H | |
| 11 | $-CH_3$ | $-OCH_3$ | H | H | H | $-COCH_3$ | |
| 12 | 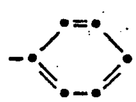 | Cl | H | H | H | $-COCH_3$ | |
| 13 | 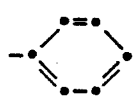 | Cl | H | H | H | H | |

## Beispiel 2: Insektizide Kontaktwirkung: Aphis craccivora

In Töpfen angezogene Pflanzen (Vicia faba) werden vor dem Versuchsbeginn mit je ca. 200 Individuen der Spezies Aphis craccivora besiedelt. Die so behandelten Pflanzen werden 24 Stunden später mit einer Lösung enthaltend 3, 12,5 , 50 oder 100 ppm der zu prüfenden Verbindung bis zur Tropfnässe besprüht. Man verwendet pro Test-Verbindung und pro Konzentration zwei Pflanzen, und eine Auswertung der erzielten Abtötungsrate erfolgt nach weiteren 24 Stunden.

Die Verbindungen gemäss Beispiel 1 zeigen im obigen Versuch die in der folgenden Tabelle angegebene Wirkung gegen Insekten der Spezies Aphis craccivora.

Beispiel 3: Insektizide Wirkung (systemisch): Aphis craccivora

Bewurzelte Bohnenpflanzen werden in Töpfe, welche 600 ccm Erde enthalten, verpflanzt und anschliessend 50 ml einer Lösung der zu prüfenden Verbindung (erhalten aus einem 25%igen Spritzpulver) in einer Konzentration von 3, 12,5 , 50 oder 100 ppm direkt auf die Erde aufgegossen.

Nach 24 Stunden werden auf die oberirdischen Pflanzenteile Läuse der Spezies Aphis craccivora gesetzt und die Pflanzen mit einem Plastikzylinder überstülpt, um die Läuse vor einer eventuellen Kontakt- oder Gaswirkung der Testsubstanz zu schützen.

Die Auswertung der erzielten Abtötung erfolgt 48 und 72 Stunden nach Versuchsbeginn. Pro Konzentrationsdosis Testsubstanz werden zwei Pflanzen, je eine in einem separaten Topf, verwendet. Der Versuch wird bei 25°C und 70% relativer Luftfeuchtigkeit durchgeführt.

Die Verbindungen gemäss Beispiel 1 zeigen im obigen Versuch die in der folgenden Tabelle angegebene systemische Wirkung gegen Insekten der Spezies Aphis craccivora.

Biologische Versuchsergebnisse

In der folgenden Tabelle sind Versuchsergebnisse auf der Basis der vorstehenden Beispiele aufgeführt, und zwar mit folgendem Bewertungsindex in bezug auf die prozentuale Abtötung der Schädlinge.

A : 70-100% Abtötung bei 3 ppm Wirkstoffkonzentration.

B : 70-100% Abtötung bei 12,5 ppm Wirkstoffkonzentration.

C : 70-100% Abtötung bei 50 ppm Wirkstoffkonzentration.

D : 70-100% Abtötung bei 100 ppm Wirkstoffkonzentration.

| Verb. Nr. | Kontaktwirkung gegen Aphis craccivora | Systemische Wirkung gegen Aphis craccivora |
|---|---|---|
| 1 | A | A |
| 2 | B | C |
| 3 | B | B |
| 4 | B | B |
| 5 | A | A |
| 6 | A | B |
| 7 | B | B |
| 8 | A | B |
| 9 | A | B |

Patentansprüche

1. Die Verwendung einer Verbindung der Formel I

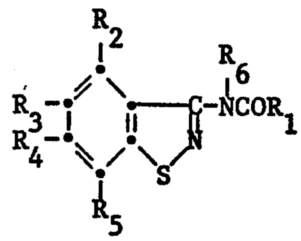

(I)

worin $R_1$ gegebenenfalls substituiertes Alkyl oder Phenyl $R_2$, $R_3$, $R_4$ und $R_5$ unabhängig voneinander Wasserstoff, Halogen, $C_1$-$C_5$-Alkyl, $C_1$-$C_5$-Alkoxy, Trifluormethyl, Amino oder Nitro, und $R_6$ Wasserstoff oder $-COR_1$ bedeuten, zur Bekämpfung von verschiedenartigen Schädlingen an Tieren und Pflanzen.

2. Die Verwendung gemäss Anspruch 1 zur Bekämpfung von Insekten und Vertretern der Ordnung Akarina.

3. Ein Schädlingsbekämpfungsmittel, welches als aktive Komponente eine Verbindung der Formel

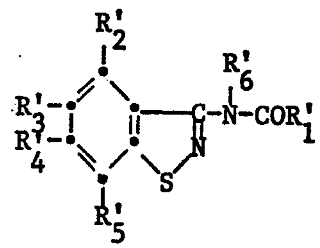

(Ia)

enthält, worin

$R'_1$ durch Halogen oder $C_1$-$C_5$-Alkoxy substituiertes $C_1$-$C_{10}$ Alkyl oder unsubstituiertes $C_2$-$C_{10}$-Alkyl oder Phenyl und

$R'_2$, $R'_3$, $R'_4$ und $R'_5$ unabhängig voneinander Wasserstoff, Halogen, $C_1$-$C_5$-Alkyl, $C_1$-$C_5$-Alkoxy, Trifluormethyl, Amino oder Nitro und $R'_6$ Wasserstoff oder $-COR'_1$ oder

$R'_1$ Methyl, $R'_2$, $R'_3$, $R'_4$ und $R'_5$ unabhängig voneinander Wasserstoff, Halogen, $C_1$-$C_5$-Alkyl, $C_1$-$C_5$-Alkoxy, Trifluormethyl, Amino oder Nitro und $R'_6$ Wasserstoff oder

$R_1'$ Methyl, $R_2'$ Wasserstoff, $C_1$-$C_5$-Alkyl, $C_1$-$C_5$-Alkoxy, Trifluormethyl, Amino oder Nitro, $R_3'$, $R_4'$ und $R_5'$ unabhängig voneinander Wasserstoff, Trifluormethyl, Amino oder Nitro und $R_6'$ $-COR_1'$ bedeuten.

4. Ein Mittel gemäss Anspruch 3, welches als aktive Komponente eine Verbindung der Formel Ia enthält, worin
$R_1'$ durch Fluor oder Chlor substituiertes $C_1$-$C_5$-Alkyl oder unsubstituiertes $C_2$-$C_5$-Alkyl und
$R_2'$, $R_3'$, $R_4'$ und $R_5'$ unabhängig voneinander Wasserstoff, Chlor, Methyl, Trifluormethyl, Amino oder Nitro und $R_6'$ Wasserstoff oder $-COR_1'$ oder
$R_1'$ Methyl,
$R_2'$, $R_3'$, $R_4'$ und $R_5'$ unabhängig voneinander Wasserstoff, Chlor, Methyl, Trifluormethyl, Amino oder Nitro und $R_6'$ Wasserstoff oder
$R_1'$ Methyl, $R_2'$ Wasserstoff, $C_1$-$C_5$-Alkyl, $C_1$-$C_5$-Alkoxy, Trifluormethyl, Amino oder Nitro, $R_3'$, $R_4'$ und $R_5'$ unabhängig voneinander Wasserstoff, Chlor, Methyl, Trifluormethyl, Amino oder Nitro und $R_6'$ $-COR_1$ bedeuten.

5. Ein Mittel gemäss Anspruch 4, welches als aktive Komponente die Verbindung der Formel

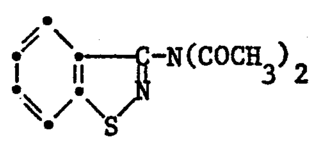

enthält.

6. Ein Mittel gemäss Anspruch 4, welches als aktive Komponente die Verbindung der Formel

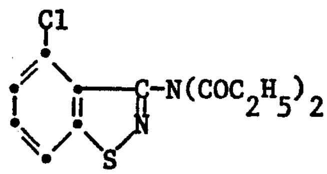

enthält.

7. Ein Mittel gemäss Anspruch 4, welches als aktive Komponente die Verbindung der Formel

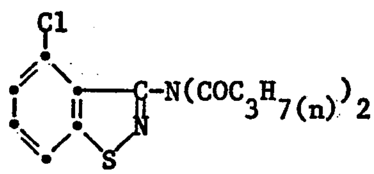

enthält.

8. Ein Mittel gemäss Anspruch 4, welches als aktive Komponente die Verbindung der Formel

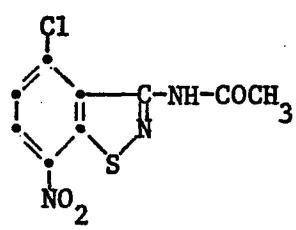

enthält.

9. Ein Mittel gemäss Anspruch 4, welches als aktive Komponente die Verbindung der Formel

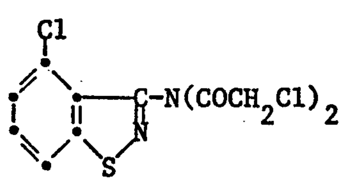

enthält.

10. Ein Mittel gemäss Anspruch 4, welches als aktive Komponente die Verbindung der Formel

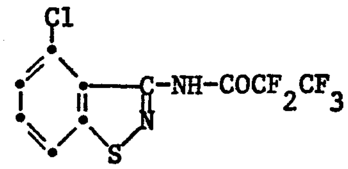

enthält.

11. Ein Mittel gemäss Anspruch 4, welches als aktive Komponente die Verbindung der Formel

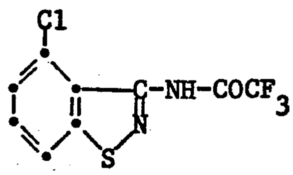

enthält.

12. Ein Mittel gemäss Anspruch 4, welches als aktive Komponente die Verbindung der Formel

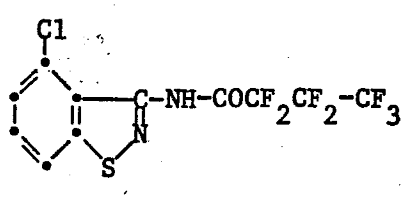

enthält.

13. Ein 3-Acylamino-benzisothiazol der Formel

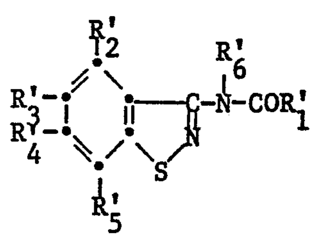

(Ia)

worin

$R_1'$ durch Halogen oder $C_1$-$C_5$-Alkoxy substituiertes $C_1$-$C_{10}$ Alkyl oder unsubstituiertes $C_2$-$C_{10}$-Alkyl oder Phenyl und

$R_2'$, $R_3'$, $R_4'$ und $R_5'$ unabhängig voneinander Wasserstoff, Halogen,

$C_1$-$C_5$-Alkyl, $C_1$-$C_5$-Alkoxy, Trifluormethyl, Amino oder Nitro und $R_6'$ Wasserstoff oder $-COR_1'$ oder $R_1'$ Methyl, $R_2'$, $R_3'$, $R_4'$ und $R_5'$ unabhängig voneinander Wasserstoff, Halogen, $C_1$-$C_5$-Alkyl, $C_1$-$C_5$-Alkoxy, Trifluormethyl, Amino oder Nitro und $R_6'$ Wasserstoff oder $R_1'$ Methyl, $R_2'$ Wasserstoff, $C_1$-$C_5$-Alkyl, $C_1$-$C_5$-Alkoxy, Trifluormethyl, Amino oder Nitro, $R_3'$, $R_4'$ und $R_5'$ unabhängig voneinander Wasserstoff, Trifluormethyl, Amino oder Nitro und $R_6'$ $-COR_1'$ bedeuten.

14. Eine Verbindung gemäss Anspruch 13, worin $R_1'$ durch Fluor oder Chlor substituiertes $C_1$-$C_5$-Alkyl oder unsubstituiertes $C_2$-$C_5$-Alkyl, und $R_2'$, $R_3'$, $R_4'$ und $R_5'$ unabhängig voneinander Wasserstoff, Chlor, Methyl, Trifluormethyl, Amino oder Nitro und $R_6'$ Wasserstoff oder $-COR_1'$ oder $R_1'$ Methyl, $R_2'$, $R_3'$, $R_4'$ und $R_5'$ unabhängig voneinander Wasserstoff, Chlor, Methyl, Trifluormethyl, Amino oder Nitro und $R_6'$ Wasserstoff oder $R_1'$ Methyl, $R_2'$ Wasserstoff, $C_1$-$C_5$-Alkyl, $C_1$-$C_5$-Alkoxy, Trifluormethyl, Amino oder Nitro, $R_3'$, $R_4'$ und $R_5'$ unabhängig voneinander Wasserstoff, Chlor, Methyl, Trifluormethyl, Amino oder Nitro und $R_6'$ $-COR_1$ bedenken.

15. Die Verbindung gemäss Anspruch 14 der Formel

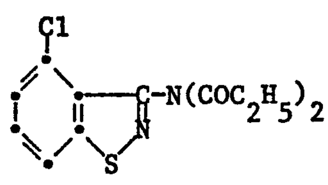

16. Die Verbindung gemäss Anspruch 14 der Formel

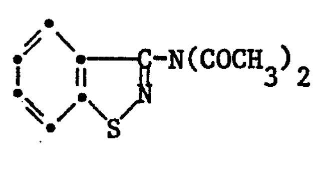

17. Die Verbindung gemäss Anspruch 14 der Formel

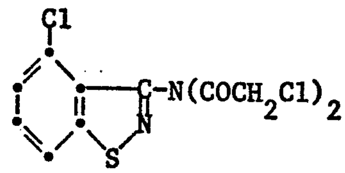

.

18. Die Verbindung gemäss Anspruch 14 der Formel

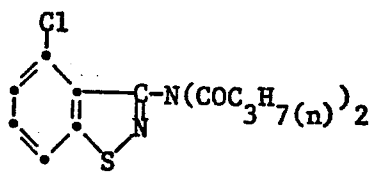

.

19. Die Verbindung gemäss Anspruch 14 der Formel

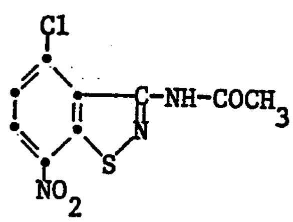

.

20. Die Verbindung gemäss Anspruch 14 der Formel

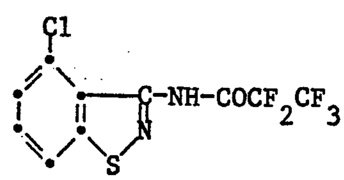

.

21. Die Verbindung gemäss Anspruch 14 der Formel

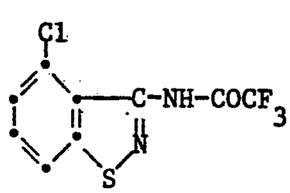

.

22. Die Verbindung gemäss Anspurch 14 der Formel

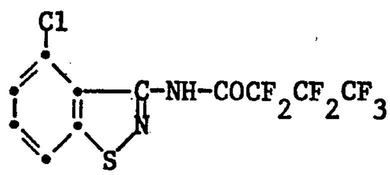

.

23. Verfahren zur Herstellung von Verbindungen gemäss Anspruch 13 der Formel Ia, dadurch gekennzeichnet, dass man eine Verbindung der Formel

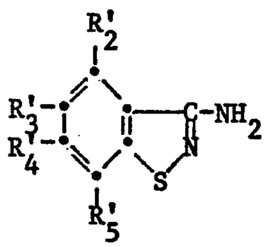

mit einer Verbindung der Formel

$ClCOR'_1$ oder 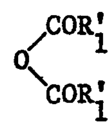

umsetzt, worin $R'_1$ bis $R'_5$ die im Anspruch 13 angegebene Bedeutung haben.

FO 7.5/WH/mg*